(19) Europäisches Patentamt
European Patent Office
Office européen des brevets

(11) Publication number: **0 387 790 A2**

(12) **EUROPEAN PATENT APPLICATION**

(21) Application number: **90104726.6**

(22) Date of filing: **13.03.90**

(51) Int. Cl.[5]: **C07C 47/21, C07C 59/74, C07C 47/263, C07C 57/13, C07C 59/42**

(30) Priority: **13.03.89 JP 60324/89**

(43) Date of publication of application: **19.09.90 Bulletin 90/38**

(84) Designated Contracting States: **DE FR GB**

(71) Applicant: **DAIKIN INDUSTRIES, LIMITED**
**Umeda Center Building 4-12 Nakazaki-nishi**
**2-chome Kita-ku**
**Osaka 530(JP)**

(72) Inventor: **Kuwahara, Yasumasa**
**208-102 Namiki 2-chome**
**Tsukuba-shi, Ibaraki-ken(JP)**

(74) Representative: **Hansen, Bernd, Dr.rer.nat. et al**
**Hoffmann, Eitle & Partner Patentanwälte**
**Arabellastrasse 4 Postfach 81 04 20**
**D-8000 München 81(DE)**

(54) **Novel monoterpenes.**

(57) Monoterpenes of the formula:

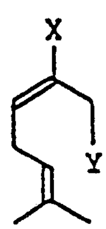

wherein X and Y are independently -CHO, $-CO_2H$ or $-CH_2OR$, R being hydrogen or tetrahydropyranyl have been isolated from agriculturally harmful acarids and then identified. They have fragrance of citrus-note and are bactericidally active against bacteria which may cause crop injury. In addition, they are the first sex pheromones to be isolated from the acarids. A composition containing said monoterpenes and the use of said monoterpenes or of the composition as bactericide and/or sex pheromone is also disclosed.

EP 0 387 790 A2

# NOVEL MONOTERPENES

## BACKGROUND OF THE INVENTION

### Field of the Invention

The present invention relates to novel monoterpenes, compositions containing these monoterpenes and their use as bactericide and/or sex pheromone.

## DESCRIPTION OF THE RELATED ART

Monoterpenes are hydrocarbons having a basal structure of $C_{10}H_{16}$. Most of them are naturally found in essential oils. The monoterpenes have been utilized, for example, in an insecticide, medicine or perfume due to their volatility and fragrance.

## SUMMARY OF THE INVENTION

Novel monoterpenes have been isolated from agriculturally harmful acarids and then identified. They have fragrance of citrus-note and are bactericidally active against bacteria which may cause crop injury. In addition, they are the first sex pheromones to be isolated from the acarids.

Accordingly, the present invention provides monoterpenes of the formula:

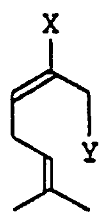

wherein X and Y are independently -CHO, $-CO_2H$ or $-CH_2OR$, R being hydrogen or tetrahydropyranyl.

## DETAILED DESCRIPTION OF THE INVENTION

The present monoterpenes can be obtained from acarids such as Tyrophagus putrescentiae and Caloglyphus polyphyllae.

The present monoterpenes have fragrance of citrus-note and bactericidal activity against some bacteria which may harm crops.

In addition, the monoterpenes are the first sex pheromones that have been isolated from genus acarid, which attract male Caloglyphus polyphyllae. Therefore, it is possible to use the monoterpenes as an attractant.

Specific examples of the present monoterpenes as well as their characteristic data are shown as follows:

2(E)-(4-methyl-3-pentenylidene)-butanedial (hereinafter referred to as β-acaridial):

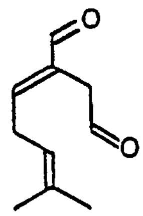

MS: 166.0988 ($M^+$), M/Z = 151, 137, 123
IR ($CCl_4$): 2810, 2700, 1720, 1680 $cm^{-1}$
UV (hexane): $\lambda_{max}$ = 225 nm, $\epsilon$ = 13600
NMR (500 MHz, $CDCl_3$): $\delta$ =9.45 (s), 9.62 (t, J=1.6 Hz), 6.75 (t, J=7.44 Hz), 5.12 (triplet-quintet, J=7.25, 7.1, 1.28 Hz), 3.43 (2H, d, J=1.3 Hz), 3.00 (2H, t, J=7.34 Hz), 1.73 (3H, s), 1.65 (3H, s)
2(E)-(4-methyl-3-pentenylidene)-butanediol (hereinafter referred to as $\beta$-acaridiol):

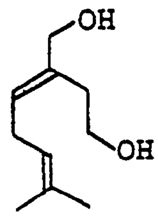

$^{13}C$-NMR: $\delta$ = 135.97 (C), 132.47 (C), 130.02 (CH), 121.99 (CH), 61.80 ($CH_2$), 58.45 ($CH_2$), 32.38 ($CH_2$), 26.82 ($CH_2$), 25.67 ($CH_3$), 17.77 ($CH_3$)

The present monoterpenes can be obtained as follows by the isolation from the acarids or the synthesis:

Isolation from acarids:

Tyrophagus putrescentiae was cultured and extracted with n-hexane. The extract was subjected to silica column chromatography and eluted with n-hexane/ether. $\beta$-Acaridial was obtained in fractions of 90:10-80:20.

Synthesis:

The present monoterpenes can be obtained according to the following reaction formulae:

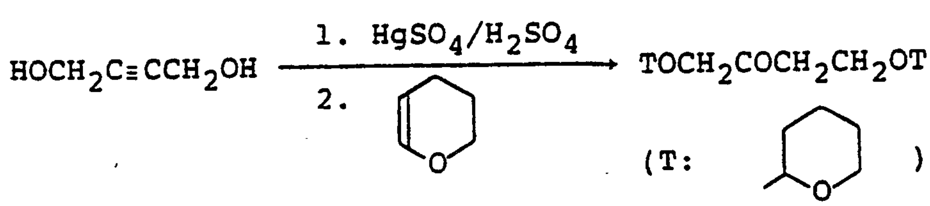

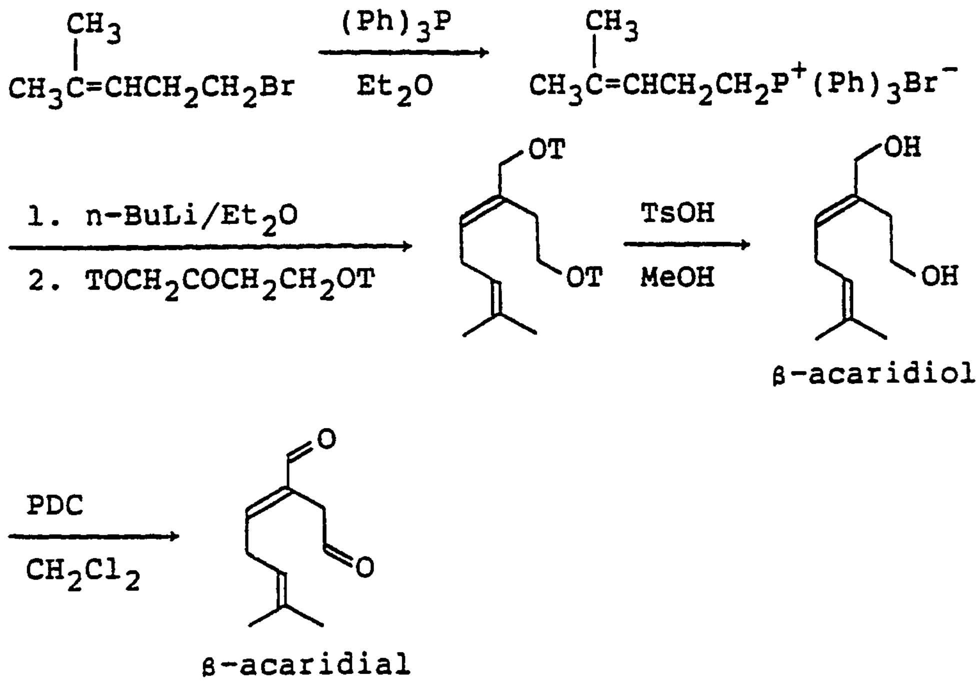


β-acaridiol

β-acaridial

## PREFERRED EMBODIMENTS OF THE INVENTION

### Example 1

Bactericidal activity of β-acaridial

By using a paper disc for antibiotics assay (diameter: 8 cm), the bactericidal activity of β-acaridial was examined against the bacteria shown in Table 1.

In the examination, a usual agar medium was used as a culture medium for the bacteria. A solution of the test compound in ethanol was applied on the disc so as to inhibit the bacteria. The bactericidal activity was determined as a diameter of the inhibited area.

The results as well as the concentration of the ethanol solution of the test compound are shown in Table 1.

Table 1

| Bactericidal activity of β-acaridial | | | |
|---|---|---|---|
| Concentration (μm/ml) | Inhibition diameter (mm) | | |
| | A | P | F |
| 500 | 8.0 | 14.3 | 11.5 |
| 400 | - | 12.0 | 10.3 |
| 320 | - | 11.6 | 9.7 |
| 256 | - | 11.1 | 9.5 |
| 205 | - | 11.1 | 8.8 |
| 164 | - | 9.6 | 8.6 |
| 131 | - | 9.4 | 8.4 |
| 105 | - | 9.3 | 8.0 |
| 84 | - | 8.0 | - |
| A: Alternaria alternata<br>P: Penicillium Vermicalatum IFO 7231<br>F: Fusarium Oxysporum | | | |

Example 2

Attraction of β-acaridial for male Caloglyphus polyphyllae

Into a dish (diameter: 4 cm) containing a small amount of dry yeast on the center thereof and a moist piece of filter paper placed aside from the center, ten head of male Caloglyphus polyphyllae were introduced. The moment the acarids began to eat the yeast, two pieces of filter paper were put on the dish symmetrically about the center, one piece containing a solution of the test compound and the other piece only a solvent as a control. The distance between the center of the dish and each piece was 1 cm. The attraction was concluded to be positive when the acarid stopped to eat and moved or were headed toward the test piece.

The positive attraction was achieved with the minimal concentration of β-acaridial of 1 ppm, which is about a half of the β-acaridial concentration in one head of female Caloglyphus polyphyllae.

## Claims

1. Monoterpenes of the formula:

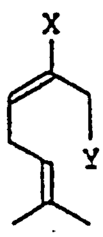

wherein X and Y are independently -CHO, $-CO_2H$ or $-CH_2OR$, R being hydrogen or tetrahydropyranyl.

2. A composition, which contains one or more monoterpenes according to claim 1 together with a usual carrier and/or diluent.

3. The use of a compound or composition according to claims 1 or 2 as bactericide and/or as sex pheromone.